# EUROPEAN PATENT APPLICATION

(11) **EP 4 091 450 A1**
(43) Date of publication of application: **23.11.2022**
(21) Application number: 21174857.9
(22) Date of filing: 19.05.2021
(51) Int. Cl.: A01N 43/38, C07D 209/34, A01N 43/90, C07D 471/04

(54) **3,3-DIFLUORO-2-OXOINDOLINE DERIVATIVES USEFUL IN WEED CONTROL**

(71) Applicant: Syngenta Crop Protection AG, 4058 Basel (CH)
(72) Inventor: The designation of the inventor has not yet been filed
(74) Representative: SYNGENTA IP

(57) **Abstract**

The present invention relates to the use of a compound of Formula (I), 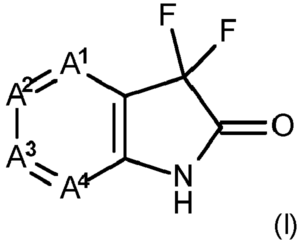 wherein A1, A2, A3 and A4 are as defined herein or an agronomically acceptable salt of said compound as a herbicide. The invention further relates to herbicidal compositions which comprise a compound of Formula (I) and to the use of compounds of Formula (I) for controlling weeds, in particular in crops of useful plants.

## Description

The present invention relates to the use of certain compounds as herbicides, to herbicidal compositions which comprise the compounds, and to their use for controlling weeds, in particular in crops of useful plants, or for inhibiting plant growth.

The present invention is based on the finding that certain difluoro cyclic amides of formula (I) as defined herein, exhibit surprisingly good herbicidal activity. Thus, according to the present invention there is provided the use of a compound of Formula (I), 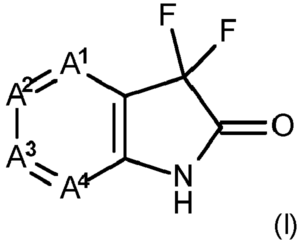 Wherein
the ring comprising A1, A2, A3 and A4 together with the carbon atoms of the adjacent ring to which A1 and A4 are attached is aromatic;
A1 is selected from the group consisting of C-R1 and nitrogen;
A2 is selected from the group consisting of C-R2 and nitrogen;
A3 is selected from the group consisting of C-R3 and nitrogen;
A4 is selected from the group consisting of C-R4 and nitrogen;
at most two of A1, A2, A3 and A4 are nitrogen; and
each R1, R2, R3 and R4 is independently selected from the group consisting of hydrogen, halogen, amino, hydroxyl, C1-C5alkyl, C3-4cycloalkyl, C1-C4alkoxy, C2-C3alkenyl, C2-C3alkynyl, C1-C2haloalkoxy, halophenyl, C1-2haloalkyl, C1-C2alkylsulfanyl, C1-C2 alkylsulphinyl and C1-C2alkylsulfonyl and cyano;
or an agronomically acceptable salt of said compound, as a herbicide.

According to a second aspect of the invention, there is provided an agrochemical composition comprising a herbicidally effective amount of a compound of formula (I) and an agrochemically-acceptable diluent or carrier. Such an agricultural composition may further comprise at least one additional active ingredient.

According to a third aspect of the invention, there is provided a method of controlling or preventing undesirable plant growth, wherein a herbicidally effective amount of a compound of formula (I), or a composition comprising this compound as active ingredient, is applied to the plants, to parts thereof or the locus thereof.

As used herein, the term "halogen" or "halo" refers to fluorine (fluoro), chlorine (chloro), bromine (bromo) or iodine (iodo), preferably fluorine, chlorine or bromine.

As used herein, cyano means a -CN group.

As used herein, hydroxy or hydroxyl means an -OH group.

As used herein, amino means an -NH₂ group.

As used herein, the term "C₁-C₅alkyl" refers to a straight or branched hydrocarbon chain radical consisting solely of carbon and hydrogen atoms, containing no unsaturation, having from one to five carbon atoms, and which is attached to the rest of the molecule by a single bond. C₁-C₃alkyl and C₁-C₂alkyl are to be construed accordingly. Examples of C₁-Csalkyl include, but are not limited to, methyl (Me), ethyl (Et), n-propyl, 1-methylethyl (isopropyl), n-butyl, and 1-dimethylethyl (*t*-butyl).

As used herein, the term "C₁-C₄alkoxy" refers to a radical of the formula -ORₐ where Rₐ is a C₁-C₄alkyl radical as generally defined above. C₁-C₂alkoxy is to be construed accordingly. Examples of C₁-₄alkoxy include, but are not limited to, methoxy, ethoxy, propoxy, iso-propoxy and t-butoxy.

As used herein, the term "C₁-C₂haloalkyl" refers to a C₁-C₂alkyl radical as generally defined above substituted by one or more of the same or different halogen atoms. C₂haloalkyl is to be construed accordingly. Examples of C₁-C₂haloalkyl include, but are not limited to chloromethyl, fluoromethyl, fluoroethyl, difluoromethyl, trifluoromethyl and 2,2,2-trifluoroethyl.

As used herein, the term "C₂-C₃alkenyl" refers to a straight or branched hydrocarbon chain radical group consisting solely of carbon and hydrogen atoms, containing at least one double bond that can be of either the (*E*)- or (Z)-configuration, having from two to three carbon atoms, which is attached to the rest of the molecule by a single bond. Examples of C₂-C₃alkenyl include, but are not limited to ethenyl, prop-1-enyl and allyl (prop-2-enyl).

As used herein, the term "C₂-C₃alkynyl" refers to a straight or branched hydrocarbon chain radical group consisting solely of carbon and hydrogen atoms, containing at least one triple bond, having from two to three carbon atoms, and which is attached to the rest of the molecule by a single bond. Examples of C₂-C₃alkynyl include, but are not limited to ethynyl, prop-1-ynyl and propargyl (prop-2-ynyl).

As used herein, the term "C₁-C₂haloalkoxy" refers to a C₁-C₂alkoxy group as defined above substituted by one or more of the same or different halogen atoms. Examples of C₁-C₂haloalkoxy include, but are not limited to fluoromethoxy, difluoromethoxy, fluoroethoxy, trifluoromethoxy and trifluoroethoxy.

As used herein, the term "C₁₋₂alkylsulfanyl" refers to a radical of the formula - SRₐ wherein Rₐ is a C₁₋₂alkyl radical as generally defined above.

As used herein, the term "C₁₋₂alkylsulfinyl" refers to a radical of the formula - S(O)Rₐ wherein Rₐ is a C₁₋₂alkyl radical as generally defined above.

As used herein, the term "C₁₋₂alkylsulfonyl" refers to a radical of the formula - S(O)₂Rₐ wherein Rₐ is a C₁₋₂alkyl radical as generally defined above.

As used herein, the term "C₃₋₄cycloalkyl" refers to a stable, monocyclic ring radical which is saturated and contains 3 to 4 carbon atoms. Examples of C₃₋₄cycloalkyl include, but are not limited to, cyclopropyl and cyclobutyl.

The compounds of formula (I) will typically be provided in the form of an agronomically acceptable salt, a zwitterion or an agronomically acceptable salt of a zwitterion. This invention covers all such agronomically acceptable salts, zwitterions and mixtures thereof in all proportions.

The following list provides definitions, including preferred definitions, for substituents A1, A2, A3, A4, R1, R2, R3 and R4 with reference to the compounds of formula (I) according to the invention. For any one of these substituents, any of the definitions given below may be combined with any definition of any other substituent given below or elsewhere in this document.

Preferably A1 is C-R1. Preferably R1 is selected from the group consisting of hydrogen, halogen, C3-4cycloalkyl and cyano, more preferably hydrogen, halogen and cyclopropyl, most preferably hydrogen and chlorine.

Preferably A2 is C-R2. Preferably R2 is selected from the group consisting of hydrogen, halogen, C3-4cycloalkyl and cyano, more preferably hydrogen, halogen and cyclopropyl, most preferably hydrogen and chlorine.

Preferably A3 is C-R3. Preferably R3 is selected from the group consisting of hydrogen, halogen, C3-4cycloalkyl and cyano, more preferably hydrogen, halogen and cyclopropyl, most preferably hydrogen and chlorine.

Preferably A4 is C-R4. Preferably R4 is selected from the group consisting of hydrogen, halogen, C3-4cycloalkyl and cyano, more preferably hydrogen, halogen and cyclopropyl, most preferably hydrogen and chlorine.
A preferred subset of compounds is one in which;
A1 is selected from the group consisting of C-R1 and nitrogen;
A2 is selected from the group consisting of C-R2;
A3 is selected from the group consisting of C-R3 and nitrogen;
A4 is selected from the group consisting of C-R4; and
each R1, R1, R1 and R1 is independently selected from the group consisting of hydrogen, halogen, C3-4cycloalkyl and cyano.

A more preferred subset of compounds is one in which;
A1 is selected from the group consisting of C-R1 and nitrogen;
A2 is selected from the group consisting of C-R2;
A3 is selected from the group consisting of C-R3;
A4 is selected from the group consisting of C-R4; and
each R1, R1, R1 and R1 is independently selected from the group consisting of hydrogen, halogen, cyclopropyl.

### Table of Examples

Table 1 below discloses 402 compounds designated compound numbers 1-1 to 1-402 respectively, of formula (I), wherein the values of A1-A4 are as given in Table 1.

**Table 1**

| **Compound Number** | **A¹** | **A²** | **A³** | **A⁴** |
|---|---|---|---|---|
| 1-1 | CH | CH | CH | CH |
| 1-2 | CH | CH | CF | CH |
| 1-3 | CH | CH | CCl | CH |
| 1-4 | CH | CH | CBr | CH |
| 1-5 | CH | CH | CMe | CH |
| 1-6 | CH | CH | CCF3 | CH |
| 1-7 | CH | CH | COCF3 | CH |
| 1-8 | CH | CH | CH | CF |
| 1-9 | CH | CH | CH | CCl |
| 1-10 | CH | CH | CH | CBr |
| 1-11 | CH | CH | CH | CMe |
| 1-12 | CH | CH | CH | CEt |
| 1-13 | CH | CH | CH | CCF3 |
| 1-14 | CH | CH | CH | CCH=CH2 |
| 1-15 | CH | CH | CH | CCCH |
| 1-16 | CH | CH | CH | COMe |
| 1-17 | CH | CH | CH | CSMe |
| 1-18 | CH | CH | CH | CSOMe |
| 1-19 | CH | CH | CH | CSO2Me |
| 1-20 | CH | CF | CH | CH |
| 1-21 | CH | CF | CF | CH |
| 1-22 | CH | CF | CCl | CH |
| 1-23 | CH | CF | CBr | CH |
| 1-24 | CH | CF | CMe | CH |
| 1-25 | CH | CF | CCF3 | CH |
| 1-26 | CH | CF | CH | CF |
| 1-27 | CH | CF | CH | CCl |
| 1-28 | CH | CCl | CH | CH |
| 1-29 | CH | CCl | CF | CH |
| 1-30 | CH | CCl | CCl | CH |
| 1-31 | CH | CCl | CBr | CH |
| 1-32 | CH | CCl | CMe | CH |
| 1-33 | CH | CCl | CCF3 | CH |
| 1-34 | CH | CCl | CH | CF |
| 1-35 | CH | CCl | CH | CCl |
| 1-36 | CH | CBr | CH | CH |
| 1-37 | CH | CCN | CH | CH |
| 1-38 | CH | N | CH | CH |
| 1-39 | CH | N | CF | CH |
| 1-40 | CH | N | CCl | CH |
| 1-41 | CH | N | CBr | CH |
| 1-42 | CH | N | CMe | CH |
| 1-43 | CH | N | CCF3 | CH |
| 1-44 | CH | N | CH | CF |
| 1-45 | CH | N | CH | CCl |
| 1-46 | CH | CH | N | CH |
| 1-47 | CH | CF | N | CH |
| 1-48 | CH | CCl | N | CH |
| 1-49 | CH | CH | CH | N |
| 1-50 | CH | CH | CF | N |
| 1-51 | CH | CH | CCl | N |
| 1-52 | CH | CH | CBr | N |
| 1-53 | CH | CH | CMe | N |
| 1-54 | CH | CH | CCF3 | N |
| 1-55 | CF | CH | CH | CH |
| 1-56 | CF | CH | CF | CH |
| 1-57 | CF | CH | CCl | CH |
| 1-58 | CF | CH | CBr | CH |
| 1-59 | CF | CH | CMe | CH |
| 1-60 | CF | CH | CCF3 | CH |
| 1-61 | CF | CH | CH | CF |
| 1-62 | CF | CH | CH | CCl |
| 1-63 | CF | CF | CH | CH |
| 1-64 | CF | CF | CF | CH |
| 1-65 | CF | CF | CCl | CH |
| 1-66 | CF | CF | CBr | CH |
| 1-67 | CF | CF | CMe | CH |
| 1-68 | CF | CF | CCF3 | CH |
| 1-69 | CF | CF | CH | CF |
| 1-70 | CF | CF | CH | CCl |
| 1-71 | CF | CCl | CH | CH |
| 1-72 | CF | CCl | CF | CH |
| 1-73 | CF | CCl | CCl | CH |
| 1-74 | CF | CCl | CBr | CH |
| 1-75 | CF | CCl | CMe | CH |
| 1-76 | CF | CCl | CCF3 | CH |
| 1-77 | CF | CCl | CH | CF |
| 1-78 | CF | CCl | CH | CCl |
| 1-79 | CF | N | CH | CH |
| 1-80 | CF | N | CF | CH |
| 1-81 | CF | N | CCl | CH |
| 1-82 | CF | N | CBr | CH |
| 1-83 | CF | N | CMe | CH |
| 1-84 | CF | N | CCF3 | CH |
| 1-85 | CF | N | CH | CF |
| 1-86 | CF | N | CH | CCl |
| 1-87 | CF | CH | N | CH |
| 1-88 | CF | CF | N | CH |
| 1-89 | CF | CCl | N | CH |
| 1-90 | CF | CH | CH | N |
| 1-91 | CF | CH | CF | N |
| 1-92 | CF | CH | CCl | N |
| 1-93 | CF | CH | CBr | N |
| 1-94 | CF | CH | CMe | N |
| 1-95 | CF | CH | CCF3 | N |
| 1-96 | CCl | CH | CH | CH |
| 1-97 | CCl | CH | CF | CH |
| 1-98 | CCl | CH | CCl | CH |
| 1-99 | CCl | CH | CBr | CH |
| 1-100 | CCl | CH | CMe | CH |
| 1-101 | CCl | CH | CCF3 | CH |
| 1-102 | CCl | CH | CH | CF |
| 1-103 | CCl | CH | CH | CCl |
| 1-104 | CCl | CF | CH | CH |
| 1-105 | CCl | CF | CF | CH |
| 1-106 | CCl | CF | CCl | CH |
| 1-107 | CCl | CF | CBr | CH |
| 1-108 | CCl | CF | CMe | CH |
| 1-109 | CCl | CF | CCF3 | CH |
| 1-110 | CCl | CF | CH | CF |
| 1-111 | CCl | CF | CH | CCl |
| 1-112 | CCl | CCl | CH | CH |
| 1-113 | CCl | CCl | CF | CH |
| 1-114 | CCl | CCl | CCl | CH |
| 1-115 | CCl | CCl | CBr | CH |
| 1-116 | CCl | CCl | CMe | CH |
| 1-117 | CCl | CCl | CCF3 | CH |
| 1-118 | CCl | CCl | CH | CF |
| 1-119 | CCl | CCl | CH | CCl |
| 1-120 | CCl | N | CH | CH |
| 1-121 | CCl | N | CF | CH |
| 1-122 | CCl | N | CCl | CH |
| 1-123 | CCl | N | CBr | CH |
| 1-124 | CCl | N | CMe | CH |
| 1-125 | CCl | N | CCF3 | CH |
| 1-126 | CCl | N | CH | CF |
| 1-127 | CCl | N | CH | CCl |
| 1-128 | CCl | CH | N | CH |
| 1-129 | CCl | CF | N | CH |
| 1-130 | CCl | CCl | N | CH |
| 1-131 | CCl | CH | CH | N |
| 1-132 | CCl | CH | CF | N |
| 1-133 | CCl | CH | CCl | N |
| 1-134 | CCl | CH | CBr | N |
| 1-135 | CCl | CH | CMe | N |
| 1-136 | CCl | CH | CCF3 | N |
| 1-137 | CBr | CH | CH | CH |
| 1-138 | CBr | CH | CF | CH |
| 1-139 | CBr | CH | CCl | CH |
| 1-140 | CBr | CH | CBr | CH |
| 1-141 | CBr | CH | CMe | CH |
| 1-142 | CBr | CH | CCF3 | CH |
| 1-143 | CBr | CH | CH | CF |
| 1-144 | CBr | CH | CH | CCl |
| 1-145 | CBr | CF | CH | CH |
| 1-146 | CBr | CF | CF | CH |
| 1-147 | CBr | CF | CCl | CH |
| 1-148 | CBr | CF | CBr | CH |
| 1-149 | CBr | CF | CMe | CH |
| 1-150 | CBr | CF | CCF3 | CH |
| 1-151 | CBr | CF | CH | CF |
| 1-152 | CBr | CF | CH | CCl |
| 1-153 | CBr | CCl | CH | CH |
| 1-154 | CBr | CCl | CF | CH |
| 1-155 | CBr | CCl | CCl | CH |
| 1-156 | CBr | CCl | CBr | CH |
| 1-157 | CBr | CCl | CMe | CH |
| 1-158 | CBr | CCl | CCF3 | CH |
| 1-159 | CBr | CCl | CH | CF |
| 1-160 | CBr | CCl | CH | CCl |
| 1-161 | CBr | N | CH | CH |
| 1-162 | CBr | N | CF | CH |
| 1-163 | CBr | N | CCl | CH |
| 1-164 | CBr | N | CBr | CH |
| 1-165 | CBr | N | CMe | CH |
| 1-166 | CBr | N | CCF3 | CH |
| 1-167 | CBr | N | CH | CF |
| 1-168 | CBr | N | CH | CCl |
| 1-169 | CBr | CH | N | CH |
| 1-170 | CBr | CF | N | CH |
| 1-171 | CBr | CCl | N | CH |
| 1-172 | CBr | CH | CH | N |
| 1-173 | CBr | CH | CF | N |
| 1-174 | CBr | CH | CCl | N |
| 1-175 | CBr | CH | CBr | N |
| 1-176 | CBr | CH | CMe | N |
| 1-177 | CBr | CH | CCF3 | N |
| 1-178 | CMe | CH | CH | CH |
| 1-179 | CMe | CH | CF | CH |
| 1-180 | CMe | CH | CCl | CH |
| 1-181 | CMe | CH | CBr | CH |
| 1-182 | CMe | CH | CMe | CH |
| 1-183 | CMe | CH | CCF3 | CH |
| 1-184 | CMe | CH | CH | CF |
| 1-185 | CMe | CH | CH | CCl |
| 1-186 | CMe | CF | CH | CH |
| 1-187 | CMe | CF | CF | CH |
| 1-188 | CMe | CF | CCl | CH |
| 1-189 | CMe | CF | CBr | CH |
| 1-190 | CMe | CF | CMe | CH |
| 1-191 | CMe | CF | CCF3 | CH |
| 1-192 | CMe | CF | CH | CF |
| 1-193 | CMe | CF | CH | CCl |
| 1-194 | CMe | CCl | CH | CH |
| 1-195 | CMe | CCl | CF | CH |
| 1-196 | CMe | CCl | CCl | CH |
| 1-197 | CMe | CCl | CBr | CH |
| 1-198 | CMe | CCl | CMe | CH |
| 1-199 | CMe | CCl | CCF3 | CH |
| 1-200 | CMe | CCl | CH | CF |
| 1-201 | CMe | CCl | CH | CCl |
| 1-202 | CMe | N | CH | CH |
| 1-203 | CMe | N | CF | CH |
| 1-204 | CMe | N | CCl | CH |
| 1-205 | CMe | N | CBr | CH |
| 1-206 | CMe | N | CMe | CH |
| 1-207 | CMe | N | CCF3 | CH |
| 1-208 | CMe | N | CH | CF |
| 1-209 | CMe | N | CH | CCl |
| 1-210 | CMe | CH | N | CH |
| 1-211 | CMe | CF | N | CH |
| 1-212 | CMe | CCl | N | CH |
| 1-213 | CMe | CH | CH | N |
| 1-214 | CMe | CH | CF | N |
| 1-215 | CMe | CH | CCl | N |
| 1-216 | CMe | CH | CBr | N |
| 1-217 | CMe | CH | CMe | N |
| 1-218 | CMe | CH | CCF3 | N |
| 1-219 | CCF3 | CH | CH | CH |
| 1-220 | CCF3 | CH | CF | CH |
| 1-221 | CCF3 | CH | CCl | CH |
| 1-222 | CCF3 | CH | CBr | CH |
| 1-223 | CCF3 | CH | CMe | CH |
| 1-224 | CCF3 | CH | CCF3 | CH |
| 1-225 | CCF3 | CH | CH | CF |
| 1-226 | CCF3 | CH | CH | CCl |
| 1-227 | CCF3 | CF | CH | CH |
| 1-228 | CCF3 | CF | CF | CH |
| 1-229 | CCF3 | CF | CCl | CH |
| 1-230 | CCF3 | CF | CBr | CH |
| 1-231 | CCF3 | CF | CMe | CH |
| 1-232 | CCF3 | CF | CCF3 | CH |
| 1-233 | CCF3 | CF | CH | CF |
| 1-234 | CCF3 | CF | CH | CCl |
| 1-235 | CCF3 | CCl | CH | CH |
| 1-236 | CCF3 | CCl | CF | CH |
| 1-237 | CCF3 | CCl | CCl | CH |
| 1-238 | CCF3 | CCl | CBr | CH |
| 1-239 | CCF3 | CCl | CMe | CH |
| 1-240 | CCF3 | CCl | CCF3 | CH |
| 1-241 | CCF3 | CCl | CH | CF |
| 1-242 | CCF3 | CCl | CH | CCl |
| 1-243 | CCF3 | N | CH | CH |
| 1-244 | CCF3 | N | CF | CH |
| 1-245 | CCF3 | N | CCl | CH |
| 1-246 | CCF3 | N | CBr | CH |
| 1-247 | CCF3 | N | CMe | CH |
| 1-248 | CCF3 | N | CCF3 | CH |
| 1-249 | CCF3 | N | CH | CF |
| 1-250 | CCF3 | N | CH | CCl |
| 1-251 | CCF3 | CH | N | CH |
| 1-252 | CCF3 | CF | N | CH |
| 1-253 | CCF3 | CCl | N | CH |
| 1-254 | CCF3 | CH | CH | N |
| 1-255 | CCF3 | CH | CF | N |
| 1-256 | CCF3 | CH | CCl | N |
| 1-257 | CCF3 | CH | CBr | N |
| 1-258 | CCF3 | CH | CMe | N |
| 1-259 | CCF3 | CH | CCF3 | N |
| 1-260 | N | CH | CH | CH |
| 1-261 | N | CH | CF | CH |
| 1-262 | N | CH | CCl | CH |
| 1-263 | N | CH | CBr | CH |
| 1-264 | N | CH | CMe | CH |
| 1-265 | N | CH | CCF3 | CH |
| 1-266 | N | CH | CH | CF |
| 1-267 | N | CH | CH | CCl |
| 1-268 | N | CH | CH | CBr |
| 1-269 | N | CH | CH | CMe |
| 1-270 | N | CH | CH | CEt |
| 1-271 | N | CH | CH | CCF3 |
| 1-272 | N | CH | CH | CCH=CH2 |
| 1-273 | N | CH | CH | CCCH |
| 1-274 | N | CH | CH | COMe |
| 1-275 | N | CH | CH | CSMe |
| 1-276 | N | CH | CH | CSOMe |
| 1-277 | N | CH | CH | CSO2Me |
| 1-278 | N | CF | CH | CH |
| 1-279 | N | CF | CF | CH |
| 1-280 | N | CF | CCl | CH |
| 1-281 | N | CF | CBr | CH |
| 1-282 | N | CF | CMe | CH |
| 1-283 | N | CF | CCF3 | CH |
| 1-284 | N | CF | CH | CF |
| 1-285 | N | CF | CH | CCl |
| 1-286 | N | CCl | CH | CH |
| 1-287 | N | CCl | CF | CH |
| 1-288 | N | CCl | CCl | CH |
| 1-289 | N | CCl | CBr | CH |
| 1-290 | N | CCl | CMe | CH |
| 1-291 | N | CCl | CCF3 | CH |
| 1-292 | N | CCl | CH | CF |
| 1-293 | N | CCl | CH | CCl |
| 1-294 | N | N | CH | CH |
| 1-295 | N | N | CF | CH |
| 1-296 | N | N | CCl | CH |
| 1-297 | N | N | CBr | CH |
| 1-298 | N | N | CMe | CH |
| 1-299 | N | N | CCF3 | CH |
| 1-300 | N | N | CH | CF |
| 1-301 | N | N | CH | CCl |
| 1-302 | N | CH | N | CH |
| 1-303 | N | CF | N | CH |
| 1-304 | N | CCl | N | CH |
| 1-305 | N | C-c-Pr | N | CCl |
| 1-306 | N | CH | CH | N |
| 1-307 | N | CH | CF | N |
| 1-308 | N | CH | CCl | N |
| 1-309 | N | CH | CBr | N |
| 1-310 | N | CH | CMe | N |
| 1-311 | N | CH | CCF3 | N |
| 1-312 | CEt | CH | CH | CH |
| 1-313 | CEt | CF | CH | CH |
| 1-314 | CEt | CCl | CH | CH |
| 1-315 | CEt | N | CH | CH |
| 1-316 | CEt | CH | CH | CH |
| 1-317 | CEt | CH | CF | CH |
| 1-318 | CEt | CH | CCl | CH |
| 1-319 | CEt | CH | CBr | CH |
| 1-320 | CEt | CH | CMe | CH |
| 1-321 | CEt | CH | CCF3 | CH |
| 1-322 | CEt | CH | N | CH |
| 1-323 | CEt | CH | CH | CF |
| 1-324 | CEt | CH | CH | CCl |
| 1-325 | CCH=CH2 | CH | CH | CH |
| 1-326 | CCH=CH2 | CF | CH | CH |
| 1-327 | CCH=CH2 | CCl | CH | CH |
| 1-328 | CCH=CH2 | N | CH | CH |
| 1-329 | CCH=CH2 | CH | CH | CH |
| 1-330 | CCH=CH2 | CH | CF | CH |
| 1-331 | CCH=CH2 | CH | CCl | CH |
| 1-332 | CCH=CH2 | CH | CBr | CH |
| 1-333 | CCH=CH2 | CH | CMe | CH |
| 1-334 | CCH=CH2 | CH | CCF3 | CH |
| 1-335 | CCH=CH2 | CH | N | CH |
| 1-336 | CCH=CH2 | CH | CH | CF |
| 1-337 | CCH=CH2 | CH | CH | CCl |
| 1-338 | CCCH | CH | CH | CH |
| 1-339 | CCCH | CF | CH | CH |
| 1-340 | CCCH | CCl | CH | CH |
| 1-341 | CCCH | N | CH | CH |
| 1-342 | CCCH | CH | CH | CH |
| 1-343 | CCCH | CH | CF | CH |
| 1-344 | CCCH | CH | CCl | CH |
| 1-345 | CCCH | CH | CBr | CH |
| 1-346 | CCCH | CH | CMe | CH |
| 1-347 | CCCH | CH | CCF3 | CH |
| 1-348 | CCCH | CH | N | CH |
| 1-349 | CCCH | CH | CH | CF |
| 1-350 | CCCH | CH | CH | CCl |
| 1-351 | COMe | CH | CH | CH |
| 1-352 | COMe | CF | CH | CH |
| 1-353 | COMe | CCl | CH | CH |
| 1-354 | COMe | N | CH | CH |
| 1-355 | COMe | CH | CH | CH |
| 1-356 | COMe | CH | CF | CH |
| 1-357 | COMe | CH | CCl | CH |
| 1-358 | COMe | CH | CBr | CH |
| 1-359 | COMe | CH | CMe | CH |
| 1-360 | COMe | CH | CCF3 | CH |
| 1-361 | COMe | CH | N | CH |
| 1-362 | COMe | CH | CH | CF |
| 1-363 | COMe | CH | CH | CCl |
| 1-364 | CSMe | CH | CH | CH |
| 1-365 | CSMe | CF | CH | CH |
| 1-366 | CSMe | CCl | CH | CH |
| 1-367 | CSMe | N | CH | CH |
| 1-368 | CSMe | CH | CH | CH |
| 1-369 | CSMe | CH | CF | CH |
| 1-370 | CSMe | CH | CCl | CH |
| 1-371 | CSMe | CH | CBr | CH |
| 1-372 | CSMe | CH | CMe | CH |
| 1-373 | CSMe | CH | CCF3 | CH |
| 1-374 | CSMe | CH | N | CH |
| 1-375 | CSMe | CH | CH | CF |
| 1-376 | CSMe | CH | CH | CCl |
| 1-377 | CSOMe | CH | CH | CH |
| 1-378 | CSOMe | CF | CH | CH |
| 1-379 | CSOMe | CCl | CH | CH |
| 1-380 | CSOMe | N | CH | CH |
| 1-381 | CSOMe | CH | CH | CH |
| 1-382 | CSOMe | CH | CF | CH |
| 1-383 | CSOMe | CH | CCl | CH |
| 1-384 | CSOMe | CH | CBr | CH |
| 1-385 | CSOMe | CH | CMe | CH |
| 1-386 | CSOMe | CH | CCF3 | CH |
| 1-387 | CSOMe | CH | N | CH |
| 1-388 | CSOMe | CH | CH | CF |
| 1-389 | CSOMe | CH | CH | CCl |
| 1-390 | CSO2Me | CH | CH | CH |
| 1-391 | CSO2Me | CF | CH | CH |
| 1-392 | CSO2Me | CCl | CH | CH |
| 1-393 | CSO2Me | N | CH | CH |
| 1-394 | CSO2Me | CH | CH | CH |
| 1-395 | CSO2Me | CH | CF | CH |
| 1-396 | CSO2Me | CH | CCl | CH |
| 1-397 | CSO2Me | CH | CBr | CH |
| 1-398 | CSO2Me | CH | CMe | CH |
| 1-399 | CSO2Me | CH | CCF3 | CH |
| 1-400 | CSO2Me | CH | N | CH |
| 1-401 | CSO2Me | CH | CH | CF |
| 1-402 | CSO2Me | CH | CH | CCl |

Compounds of the invention may be prepared by techniques known to the person skilled in the art of organic chemistry. General methods for the production of compounds of formula (I) are described below. Unless otherwise stated in the text, the substituents A1, A2, A3, A4, R1, R2, R3 and R4 are as defined hereinbefore. The starting materials used for the preparation of the compounds of the invention may be purchased from usual commercial suppliers or may be prepared by known methods. The starting materials as well as the intermediates may be purified before use in the next step by state of the art methodologies such as chromatography, crystallization, distillation and filtration.

Compounds of formula (I) may be prepared from nitro compounds of formula (A) as shown in reaction scheme 1.

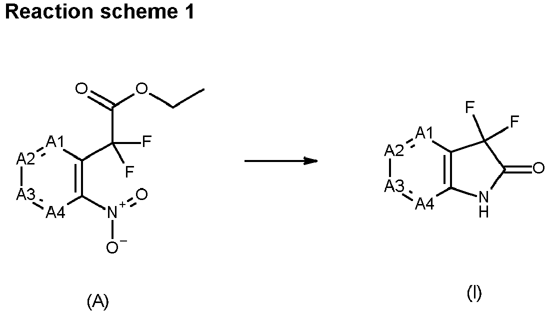

For example, a compound of formula (A) may be treated with a metal, such as iron, in a suitable solvent such as acetic acid.

Compounds of formula (A) may be prepared from aryl halides of formula (B) and alkyl halides of formula (C) as shown in reaction scheme 2.

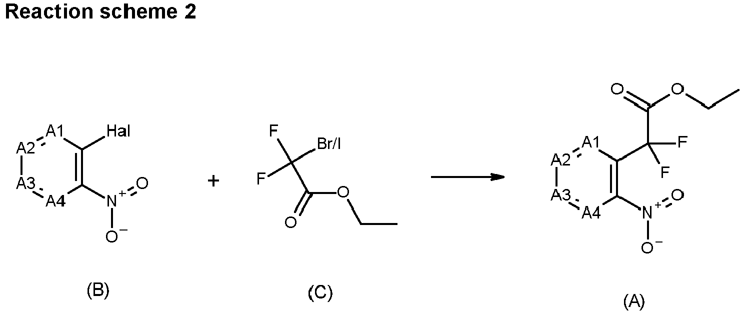

For example, a mixture of a compound of formula (B), wherein Hal represents a halogen atom, and a compound of formula (C) may be treated with a metal, such as copper, in a suitable solvent such as dimethyl sulfoxide.

Aryl halides of formula (B) are commercially available or may be prepared by methods well known in the literature.

Alkyl bromides or alkyl iodides of formula (C) are available or can be prepared by methods known in the literature.

Compounds of formula (I) may be prepared from anilines of formula (D) and alkyl halides of formula (C) as shown in reaction scheme 3.

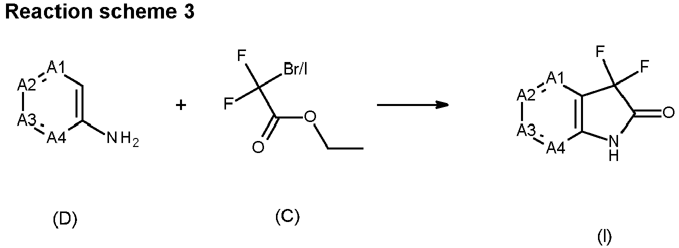

For example, a compound of formula (D) and a compound of fomula (C) can be treated with a metal catalyst, such as copper(II) acetate, optionally in the presence of a suitable ligand, such as a bipyridine ligand, for example 4,4'-di-tert-butyl-2,2'-bipyridine, and a borylating reagent, such as bis(pinacolato)diboron, and a base, such as sodium acetate, in a suitable solvent, such as 1,2-dichloroethane.

Alternatively, a compound of formula (D) and a compound of fomula (C) can be treated with a metal catalyst, such as ferrocene, in the presence of an oxidant, such as hydrogen peroxide, in a suitable solvent, such as dimethylsulfoxide.

Anilines of formula (D) are available or can be prepared by methods known in the literature.

Compounds 1-10, 1-36, 1-37 are obtainable as of the priority date of the present application from commercial sources, such as Enamine.One skilled in the art will realise that it is often possible to alter the order in which the transformations described above are conducted, or to combine them in alternative ways to prepare a wide range of compounds of formula (I). Multiple steps may also be combined in a single reaction. All such variations are contemplated within the scope of the invention.

The skilled person will also be aware that some reagents will be incompatible with certain values or combinations of the substituents A1, A2, A3, A4, R1, R2, R3 and R4, as defined herein, and any additional steps, such as protection and/or deprotection steps, which are necessary to achieve the desired transformation will be clear to the skilled person.

The compounds according to the invention can be used as herbicidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oilflowables, aqueous dispersions, oily dispersions, suspo-emulsions, capsule suspensions, emulsifiable granules, soluble liquids, water-soluble concentrates (with water or a watermiscible organic solvent as carrier), impregnated polymer films or in other forms known e.g. from the Manual on Development and Use of FAO and WHO Specifications for Pesticides, United Nations, First Edition, Second Revision (2010). For water-soluble compounds, soluble liquids, water-soluble concentrates or water soluble granules are preferred. Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alphapinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The herbicidal compositions generally comprise from 0.1 to 99 % by weight, especially from 0.1 to 95 % by weight, compounds of formula (I) and from 1 to 99.9 % by weight of a formulation adjuvant which preferably includes from 0 to 25 % by weight of a surface-active substance. The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 l/ha, especially from 10 to 1000 l/ha.

Preferred formulations can have the following compositions (weight %):

**Emulsifiable concentrates:**

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

**Dusts:**

| | |
|---|---|
| active ingredient: | 0.1 to 10 %, preferably 0.1 to 5 % |
| solid carrier: | 99.9 to 90 %, preferably 99.9 to 99 % |

**Suspension concentrates:**

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

**Wettable powders:**

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

**Granules:**

| | |
|---|---|
| active ingredient: | 0.1 to 30 %, preferably 0.1 to 15 % |
| solid carrier: | 99.5 to 70 %, preferably 97 to 85 % |

The composition of the present may further comprise at least one additional pesticide. For example, the compounds according to the invention can also be used in combination with other herbicides or plant growth regulators. In a preferred embodiment the additional pesticide is a herbicide and/or herbicide safener.

The compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bilanafos, bipyrazone, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin, ethofumesate, fenoxaprop (including fenoxaprop-P-ethyl), fenoxasulfone, fenpyrazone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, imazamox (including R-imazamox), imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate, prop-2-ynyl 4-amino-3-chloro-5-fluoro-6-(7-fluoro-1H-indol-6-yl)pyridine-2-carboxylate and cyanomethyl 4-amino-3-chloro-5-fluoro-6-(7-fluoro-1H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide, ethyl 2-[[3-[[3-chloro-5-fluoro-6-[3-methyl-2,6-dioxo-4-(trifluoromethyl)pyrimidin-1-yl]-2-pyridyl]oxy]acetate and 6-chloro-4-(2,7-dimethyl-1-naphthyl)-5-hydroxy-2-methyl-pyridazin-3-one.

The mixing partners of the compound of formula (I) may also be in the form of esters or salts, as mentioned e.g. in The Pesticide Manual, Fourteenth Edition, British Crop Protection Council, 2006.

The compound of formula (I) can also be used in mixtures with other agrochemicals such as fungicides, nematicides or insecticides, examples of which are given in The Pesticide Manual.

The mixing ratio of the compound of formula (I) to the mixing partner is preferably from 1: 100 to 1000:1.

The mixtures can advantageously be used in the above-mentioned formulations (in which case "active ingredient" relates to the respective mixture of compound of formula (I) with the mixing partner).

Compounds of formula (I) of the present invention may also be combined with herbicide safeners. Examples of such safeners include benoxacor, cloquintocet (including cloquintocetmexyl), cyprosulfamide, dichlormid, fenchlorazole (including fenchloraz oleethyl), fenclorim, fluxofenim, furilazole, isoxadifen (including isoxadifenethyl), mefenpr (incl uding mefenpyr-diethyl), metcamifen and oxabetrinil.

Particularly preferred are mixtures of a compound of formula (I) with cyprosulfamide, isoxadifen (including isoxadifen-ethyl), cloquintocet (including cloquintocet-mexyl) and/or N-(2-meth oxybenzoyl)-4-[(methyl-aminocarbonyl)amino]benzenesulfonamide.

The safeners of the compound of formula (I) may also be in the form of esters or salts, as mentioned e.g. in The Pesticide Manual, 14th Edition (BCPC), 2006. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of formula (I) to safener is from 100:1 to 1:10, especially from 20:1 to 1:1.

The mixtures can advantageously be used in the above-mentioned formulations (in which case "active ingredient" relates to the respective mixture of compound of formula (I) with the safener).

The compounds of formula (I) of this invention are useful as herbicides. The present invention therefore further comprises a method for controlling unwanted plants comprising applying to the said plants or a locus comprising them, an effective amount of a compound of the invention or a herbicidal composition containing said compound. 'Controlling' means killing, reducing or retarding growth or preventing or reducing germination. Generally the plants to be controlled are unwanted plants (weeds). 'Locus' means the area in which the plants are growing or will grow.

The rates of application of compounds of formula (I) may vary within wide limits and depend on the nature of the soil, the method of application (pre-emergence; post-emergence; application to the seed furrow; no tillage application etc.), the crop plant, the weed(s) to be controlled, the prevailing climatic conditions, and other factors governed by the method of application, the time of application and the target crop. The compounds of formula (I) according to the invention are generally applied at a rate of from 10 to 2000 g/ha, especially from 50 to 1000 g/ha. A preferred range is 10-200g/ha.

The application is generally made by spraying the composition, typically by tractor mounted sprayer for large areas, but other methods such as dusting (for powders), drip or drench can also be used.

Useful plants in which the composition according to the invention can be used include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

Crops are to be understood as also including those crops which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

Compounds of formula (I) and compositions of the invention can typically be used to control a wide variety of monocotyledonous and dicotyledonous weed species. Examples of monocotyledonous species that can typically be controlled include *Alopecurus myosuroides, Avena fatua, Brachiaria plantaginea, Bromus tectorum, Cyperus esculentus, Digitaria sanguinalis, Echinochloa crus-galli, Lolium perenne, Lolium multiflorum, Panicummiliaceum, Poa annua, Setaria viridis, Setaria faberi and Sorghum bicolor.* Examples of dicotyledonous species that can be controlled include *Abutilon theophrasti, Amaranthus retroflexus, Bidens pilosa, Chenopodium album, Euphorbia heterophylla, Galium aparine, Ipomoea hederacea, Kochia scoparia, Polygonum convolvulus, Sida spinosa, Sinapis arvensis, Solanum nigrum, Stellaria media, Veronica persica and Xanthium strumarium.*

The compounds of formula (I) are also useful for pre-harvest desiccation in crops, for example, but not limited to, potatoes, soybean, sunflowers and cotton. Pre-harvest desiccation is used to desiccate crop foliage without significant damage to the crop itself to aid harvesting.

Compounds/compositions of the invention are particularly useful in non-selective burn-down applications, and as such may also be used to control volunteer or escape crop plants.

Various aspects and embodiments of the present invention will now be illustrated in more detail by way of example. It will be appreciated that modification of detail may be made without departing from the scope of the invention.

### EXAMPLES

The Examples which follow serve to illustrate, but do not limit, the invention.

### SYNTHESIS EXAMPLES

### Example 1 Preparation of 3,3-difluoro-1H-pyrrolo[3,2-b]pyridin-2-one (Compound 1-260)

### Step 1 Synthesis of ethyl 2,2-difluoro-2-(3-nitro-2-pyridyl)acetate

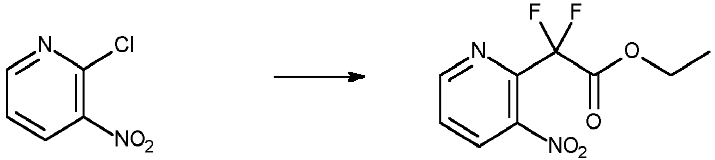

To a solution of 2-chloro-3-nitro-pyridine (1.00 g, 6.31 mmol ) in dimethylsulfoxide (10.0 mL ) were added Cu powder (3.21 g, 50.5 mmol) and ethyl 2,2-difluoro-2-iodo-acetate (1.86 mL, 12.6 mmol) . The resulting reaction mixture was stirred at 50 °C under microwave irradiation for 30 min. The reaction mixture was cooled to room temperature, diluted with water (150 mL) and extracted twice with ethyl acetate (2 x 150 mL). The combined organic layers were washed with brine (50 mL), dried over Na₂SO₄ and concentrated under reduced pressure. The crude mixture was purified by column chromatography (10 to 30% EtOAc/hexanes) to give ethyl 2,2-difluoro-2-(3-nitro-2-pyridyl)acetate (0.450 g, 1.65 mmol, 26%) as a brown liquid.
¹H NMR (400 MHz, DMSO-d6) δ 9.04 (d, 1H), 8.70 (d, 1H), 8.01 (dd, 1H), 4.38 (q, 2H), 1.23 (t, 3H) ppm

### Step 2 Synthesis of 3,3-difluoro-1 H-pyrrolo[3,2-b]pyridin-2-one

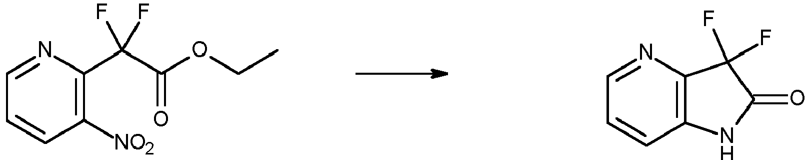

To a stirred solution of ethyl 2,2-difluoro-2-(3-nitro-2-pyridyl)acetate (18.0 g, 69.5 mmol) in acetic acid (180 mL) was added Fe (19.4 g, 347 mmol) at 25°C and stirred at 100 °C for 2 hours. The reaction was diluted with ethyl acetate (200 ml) and filtered through a pad of celite. The filtrate was concentrated in vacuo and then basified with NaHCO 3 solution and extracted twice with EtOAc (2 x 200 mL). The combined organic layers were washed with brine (100 mL), dried over Na₂SO₄ and concentrated under reduced pressure. The crude mixture was purified by column chromatography (0 to 50% EtOAc/hexanes) to give 3,3-difluoro-1H-pyrrolo[3,2-b]pyridin-2-one (5.10 g, 29.9 mmol, 43%) as yellow solid.
¹H NMR (400 MHz, DMSO-d6) δ 11.43 (s, 1H), 8.34 (d, 1H), 7.52 (m, 1H), 7.44 (d, 1H) ppm

### Example 2 Preparation of 3,3-difluoro-2-oxo-indoline-5-carbonitrile (Compound 1-37)

### Step 1 Synthesis of 3,3-difluoro-2-oxo-indoline-5-carbonitrile

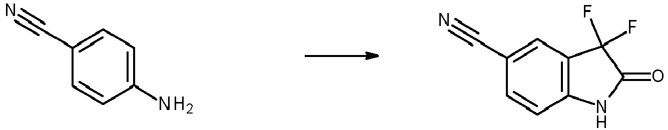

To a microwave vial were added 4-aminobenzonitrile (500 mg, 4.23 mmol) and copper(II) acetate (77 mg, 0.423 mmol) followed by 4,4'-di-tert-butyl-2,2'-bipyridine (116 mg, 0.423 mmol), bis(pinacolato)diboron (566 mg, 2.12 mmol) and sodium acetate (701 mg, 8.46 mmol). The mixture was flushed with nitrogen. Ethyl 2-bromo-2,2-difluoro-acetate (1.29 g, 6.35 mmol) and 1,2-dichloroethane (13 mL) were added to the reaction mixture and the vial was heated in the microwave at 100°C for 3 hours followed by 120°C for 2 hours. Water (30 mL) was added to the reaction mixture and it was extracted with dichloromethane (3 x 30 mL). The combined organic layers were dried over sodium sulphate, filtered and evaporated. The resultant crude product was purified using flash column chromatography (15% ethyl acetate in cyclohexane) to give 3,3-difluoro-2-oxo-indoline-6-carbonitrile (150 mg, 0.773 mmol, 18% Yield) as yellowish solid.
Mass spec. (ESI) found m/z 194.9, [M+H]⁺

### Example 3 Preparation of 3,3-difluoroindolin-2-one (Compound 1-1)

### Step 1 Synthesis of 3,3-difluoroindolin-2-one

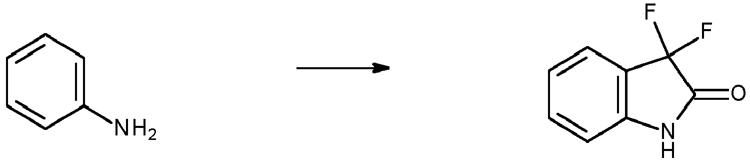

To a solution of aniline (300 mg, 3.22 mmol) in DMSO (3 mL) under an atmosphere of argon was added ferrocene (236 mg, 0.322 mmol) and ethyl 2-bromo-2,2-difluoroacetate (1.96 g, 9.66 mmol). The mixture was cooled to 0°C and a 30% hydrogen peroxide solution (730 mg, 6.44 mmol) was added drop wise. The reaction mixture was stirred at room temperature for 16 hours. Water (20 mL) was added to the reaction mixture and extracted with EtOAc (3 x 60 mL). The combined organic layers were washed with water (3 x 60 mL), brine (50 mL), dried over Na2SO4 and concentrated under reduced pressure. The resultant crude product was purified by column chromatography (eluting with 0 to 60% EtOAc/Hexane) to give 3,3-difluoroindolin-2-one (220 mg, 1.17 mmol, 36%) as a colourless liquid.
¹H NMR (400 MHz, DMSO-d6) δ 11.18 (s, 1H), 7.63 (d, 1H), 7.52 (t, 1H), 7.16 (t, 1H), 6.99 (d, 1H) ppm

### FORMULATION EXAMPLES

| **Wettable powders** | **a)** | **b)** | **c)** |
|---|---|---|---|
| active ingredients | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether (7-8 mol of ethylene oxide) | - | 2 % | - |
| highly dispersed silicic acid | 5 % | 10 % | 10 % |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

### Emulsifiable concentrate

| | |
|---|---|
| active ingredients | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| **Dusts** | **a)** | **b)** | **c)** |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill.

### Extruder granules

| | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

### Coated granules

| | |
|---|---|
| Active ingredients | 8 % |
| polyethylene glycol (mol. wt. 200) | 3 % |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed.

The obtained capsule suspension is stabilized by adding 0.25 parts of a thickener and 3 parts of a dispersing agent. The capsule suspension formulation contains 28% of the active ingredients. The medium capsule diameter is 8-15 microns.

The resulting formulation is applied to seeds as an aqueous suspension in an apparatus suitable for that purpose.

### BIOLOGICAL EXAMPLES

### Biological efficacy

Seeds of a variety of test species were sown in unsterilised compost in small pots. After cultivation for one day (pre-emergence) or seven days (post-emergence) in controlled conditions in the glasshouse (at 24/16oC, day/night; 14 hours light; 65 % humidity) the plants were sprayed with 1 mg of the active ingredient, formulated in 466µl of a acetone / water / Tween 20 (49.75:49.75:0.5) solution, which is equivalent to 1000 g/ha. Once the foliage was dry, the pots were kept in the glasshouse (at 24/16oC, day/night; 14 hours light; 65 % humidity), and were watered twice daily. After 12 days the test was evaluated and scored (100 = total damage to plant, 0 = no damage to plant). The results are shown in Table 2 below.

**Table 2**

| Compound | Rate (g/ha) | Species | | | | | | | |
|---|---|---|---|---|---|---|---|---|---|
| | | Post-Emergence | | | | Pre-Emergence | | | |
| | | AMARE | LOLPE | STEME | DIGSA | AMARE | LOLPE | STEME | DIGSA |
| 1-1 | 1000 | 30 | 0 | 0 | 10 | 0 | 0 | 0 | 0 |
| 1-10 | 1000 | 70 | 0 | 40 | 0 | 60 | 0 | 80 | 40 |
| 1-28 | 1000 | 50 | 0 | 20 | 0 | 0 | 0 | 0 | 0 |
| 1-36 | 1000 | 30 | 0 | 20 | 40 | 50 | 0 | - | 30 |
| 1-37 | 1000 | 10 | 0 | 10 | 10 | 0 | 0 | 0 | 0 |
| 1-260 | 1000 | 90 | 0 | 90 | 50 | 90 | 0 | 90 | 60 |

### Pre-emergence biological efficacy

Seeds of weeds and/or crops were sown in standard soil in pots. After cultivation for one day under controlled conditions in a glasshouse (at 24/19°C, day/night; 16 hours light), the plants were sprayed with an aqueous spray solution derived from the formulation of the technical active ingredient in a small amount of acetone and a special solvent and emulsifier mixture referred to as IF50 (11.12% Emulsogen EL360 TM + 44.44% N-methylpyrrolidone + 44.44% Dowanol DPM glycol ether), to create a 50g/l solution which was then diluted using 0.2% Genapol X080 as diluent to give the desired final dose of test compound.

The test plants were then grown under controlled conditions in the glasshouse (at 24/18°C, day/night; 15 hours light; 50 % humidity) and watered twice daily. After 13 days the test was evaluated (100 = total damage to plant; 0 = no damage to plant). The results are shown in Table 3 below.

**Table 3**

| Compound | Rate (g/ha) | Species | | | | | | | | |
|---|---|---|---|---|---|---|---|---|---|---|
| | | AMARE | STEME | ABUTH | ECHCG | ZEAMX | IPOH E | SOLNI | SETFA | LOLPE |
| 1-3 | 1000 | 70 | 70 | 60 | 50 | 0 | 30 | - | - | - |
| 1-28 | 1000 | 30 | 0 | 0 | 0 | 0 | 0 | - | - | - |
| 1-35 | 1000 | 60 | 50 | 30 | 0 | 0 | 40 | - | - | - |
| 1-96 | 1000 | 20 | 10 | 10 | 10 | 0 | 10 | - | - | - |
| 1-112 | 1000 | 50 | 40 | 10 | 10 | 0 | 0 | - | - | - |
| 1-119 | 1000 | 0 | 0 | 0 | 0 | 0 | 0 | - | - | - |
| 1-260 | 1000 | 100 | - | - | 0 | - | 50 | 100 | 40 | 10 |
| 1-305 | 1000 | 40 | 30 | 50 | 40 | 0 | 10 | - | - | - |

### Post-emergence biological efficacy

Seeds of weeds and/or crops were sown in standard soil in pots. After cultivation for 14 days under controlled conditions in a glasshouse (at 24/19°C, day/night; 16 hours light), the plants were sprayed with an aqueous spray solution derived from the formulation of the technical active ingredient in a small amount of acetone and a special solvent and emulsifier mixture referred to as IF50 (11.12% Emulsogen EL360 TM + 44.44% N-methylpyrrolidone + 44.44% Dowanol DPM glycol ether), to create a 50g/l solution which was then diluted using 0.2% Genapol XO80 as diluent to give the desired final dose of test compound.

The test plants were then grown under controlled conditions in the glasshouse (at 24/18°C, day/night; 15 hours light; 50 % humidity) and watered twice daily. After 13 days the test was evaluated (100 = total damage to plant; 0 = no damage to plant). The results are shown in Table 4 below.

**Table 4**

| Compound | Rate (g/ha) | Species | | | | | | | | |
|---|---|---|---|---|---|---|---|---|---|---|
| | | AMARE | STEME | ABUTH | ECHCG | ZEAMX | IPOHE | SOLNI | SETFA | LOLPE |
| 1-3 | 1000 | 70 | 70 | - | 40 | 20 | 50 | - | - | - |
| 1-28 | 1000 | 10 | 10 | 10 | 0 | 10 | 30 | - | - | - |
| 1-35 | 1000 | 70 | 70 | - | 10 | 10 | 60 | - | - | - |
| 1-96 | 1000 | 60 | 20 | - | 10 | 40 | 40 | - | - | - |
| 1-112 | 1000 | 50 | 20 | 10 | 20 | 10 | 10 | - | - | - |
| 1-119 | 1000 | 70 | 30 | - | 0 | 20 | 30 | - | - | - |
| 1-260 | 1000 | 100 | - | - | 0 | - | 50 | 100 | 20 | 0 |
| 1-305 | 1000 | 60 | 30 | 40 | 20 | 10 | 40 | - | - | - |

## Claims

1. Use of a compound of formula (I), 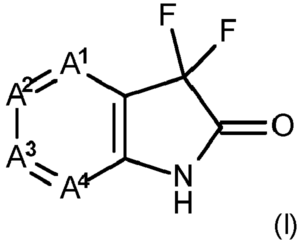 Wherein
the ring comprising A1, A2, A3 and A4 together with the carbon atoms of the adjacent ring to which A1 and A4 are attached is aromatic;
A1 is selected from the group consisting of C-R1 and nitrogen;
A2 is selected from the group consisting of C-R2 and nitrogen;
A3 is selected from the group consisting of C-R3 and nitrogen;
A4 is selected from the group consisting of C-R4 and nitrogen;
at most two of A1, A2, A3 and A4 are nitrogen; and
each R1, R2, R3 and R4 is independently selected from the group consisting of hydrogen, halogen, amino, hydroxyl, C1-C5alkyl, C3-4cycloalkyl, C1-C4alkoxy, C2-C3alkenyl, C2-C3alkynyl, C1-C2haloalkoxy, halophenyl, C1-2haloalkyl, C1-C2alkylsulfanyl, C1-C2 alkylsulphinyl and C1-C2alkylsulfonyl and cyano;
or an agronomically acceptable salt of said compound, as a herbicide.

2. Use according to claim 1, wherein in the compound of Formula (I) A2 is C-R2 and A4 is C-R4.

3. Use according to claim 1 or claim 2, wherein in the compound of Formula (I) A1 is nitrogen.

4. Use according to any one of claims 1 to 3, wherein in the compound of Formula (I) A3 is nitrogen.

5. Use according to claim 1, wherein in the compound of Formula (I) A1 is C-R1, A2 is C-R2, A3 is C-R3 and A4 is C-R4

6. Use according to any one of claims 1 to 5, wherein in the compound of Formula (I) each R1, R2, R3 and R4 is independently selected from the group consisting of hydrogen, halogen, C3-4cycloalkyl and cyano.

7. Use according to any one of claims 1 to 6, wherein in the compound of Formula (I) each R1, R2, R3 and R4 is independently selected from the group consisting of hydrogen, halogen, cyclopropyl.

8. Use according to any one of claims 1 to 7, wherein in the compound of Formula (I) one of R1, R2, R3 and R4 is halogen.

9. Use according to any one of claims 1 to 8, wherein in the compound of Formula (I) two of R1, R2, R3 and R4 are chlorine.

10. Use according to any one of claims 1 to 9, wherein in the compound of Formula (I) at least one of R1, R2, R3 and R4 is hydrogen.

11. Use according to any one of claims 1 to 7, wherein in the compound of Formula (I) each of R1, R2, R3 and R4 is hydrogen.

12. A herbicidal composition comprising (i) a compound of Formula (I) or an agronomically acceptable salt of said compound as defined in any one of claims 1 to 11 and (ii) an agriculturally acceptable formulation adjuvant.

13. A herbicidal composition according to claim 12, further comprising at least one additional pesticide.

14. A herbicidal composition according to claim 13, wherein the additional pesticide is a herbicide or herbicide safener.

15. A method of controlling weeds at a locus comprising application to the locus of a weed controlling amount of a compound of Formula (I) or an agronomically acceptable salt of said compound as defined in any one of claims 1 to 11 or a herbicidal composition according to any one of claims 12 to 14.
